# EUROPEAN PATENT SPECIFICATION

(11) **EP 1 670 436 B1**
(45) Date of publication and mention of the grant of the patent: **20.11.2013**
(21) Application number: 04768511.0
(22) Date of filing: 17.09.2004
(51) Int. Cl.: A61K 9/127

(54) **COMPOSITION COMPRISING ETHER LIPID**
ETHERLIPID ENTHALTENDE ZUSAMMENSETZUNG
COMPOSITION COMPRENANT UN LIPIDE D'ETHER

(30) Priority: 19.09.2003 GB 0322033
(43) Date of publication of application: 21.06.2006
(73) Proprietor: CAMURUS AB, 223 70 Lund (SE)
(72) Inventor: TIBERG, Fredrik, SE-223 70 Lund (SE); BARAUSKAS, Justas, SE-222 70 Lund (SE); LARSSON, Kare, SE-223 70 Lund (SE)
(74) Representative: Goddard, Christopher Robert
(86) International application number: PCT/GB2004/003965
(87) International publication number: WO 2005/027976

(56) References cited:
- WO-A-98/10747
- WO-A-99/15171
- US-A- 5 593 663
- DATABASE CA [Online] CHEMICAL ABSTRACTS SERVICE, COLUMBUS, OHIO, US; TSUTSUMI, HISAO ET AL: "Solution behavior and liquid crystalline phases of .alpha.-monoalkyl glyceryl ether/water system" XP002340650 retrieved from STN Database accession no. 101:60577 & YUKAGAKU , 33(5), 270-6 CODEN: YKGKAM; ISSN: 0513-398X, 1984,

## Description

The present invention relates to compositions suitable for the delivery of active substances. More specifically, the invention relates to compositions and precursors thereof containing lipid ethers.

The use of an appropriate formulation of an active agent can provide considerable improvements in the effectiveness of, for example, therapeutic, dietary, or cosmetic products. In pharmaceutical, veterinary and diagnostic applications, for example, active agents are often of limited solubility, limited stability, poor biological absorption and/or short retention time. An ideal composition would therefore solubilise the agent and protect it against degradation until the appropriate area of the subject (e.g. the subject's digestive tract) is reached, whereupon the agent would be released over a period that would provide an effective dose over a desired period. Similarly, dietary or cosmetic agents will typically be taken or applied only once or at most a few times per day but should remain effective over a considerable period. Different agents will require different combinations of solubility, release rate and time, protective effects and delivery properties: Formulation methods allowing control of these parameters are therefore of great value.

Amphiphile-based formulations show considerable potential in the delivery of many substances, especially for *in vivo* delivery to the human or animal body. Because the amphiphile has both polar and apolar groups which cluster to form polar and apolar regions, it can effectively solubilise both polar and apolar compounds. In addition, many of the structures formed by amphiphiles/structuring agents in polar and/or apolar solvents have a very considerable area of polar/apolar boundary at which other amphiphilic compounds can be adsorbed and stabilised. Amphiphiles can also be formulated to protect active agents, to at least some extent, from aggressive biological environments and thereby provide advantageous rates and sites of active agent release.

The formation of non-lamellar regions in the amphiphile/water, amphiphile/oil and amphiphile/oil/water phase diagrams is a well known phenomenon. Such phases include liquid crystalline phases such as the cubic P, cubic D, cubic G and hexagonal phases, which are fluid at the molecular level but show significant long-range order, and the L₃ phase which comprises a multiply interconnected bicontinuous network of bilayer sheets which are non-lamellar but lack the long-range order of the liquid crystalline phases. Depending upon their curvature, these phases may be described as normal (mean curvature towards the apolar region) or reversed (mean curvature towards the polar region).

The non-lamellar liquid crystalline and L₃ phases are thermodynamically stable systems. That is to say, they are not simply a meta-stable state that will separate and/or reform into layers, lamellar phases or the like, but are the stable thermodynamic form of the mixture.

Both lamellar and non-lamellar systems have been investigated for their properties as carriers and/or excipients for dietary, cosmetic, nutritional, diagnostic and pharmaceutical agents but the non-lamellar systems are thought to have considerable advantages in terms of their high internal surface area and bicontinuous polar and apolar regions. This has led to considerable investigation of non-lamellar phases particularly in controlled-release formulations and for solubilising relatively insoluble compounds.

As discussed above, bulk non-lamellar phase is typically a thermodynamically stable system. In addition, this bulk phase may be dispersed in a polar or non-polar solvent to form particles of a non-lamellar (especially liquid crystalline) phase in a bulk solvent. This allows the advantages of bulk non-lamellar phases to be applied in situations where use of a bulk formulation that was not miscible, with a body fluid would cause problems, such as in parenteral applications. Further control of a compound's release profile may also be achieved by such a dispersion. In many cases, the liquid crystalline or L₃ phase is in or near thermodynamic equilibrium with the excess solvent and therefore dispersions of non-lamellar particles can be prepared. Such particles may be fully (i.e. thermodynamically) stable, or may gradually degrade, thereby providing control over the release profile for active agents formulated therewith.

A method for the formation of dispersed particles of non-lamellar phase in solvents such as water is described in US 5,531,925. Such particles have a non-lamellar liquid crystalline or L₃ interior phase and a lamellar or L₃ surface phase and may also contain active ingredients.

Known particles of liquid crystalline or L₃ interior phase may be formed by methods such as adding to this phase a solution of surface-phase forming agent, stirring to form a coarse dispersion and fragmenting the resulting mixture.

In order to assess the presence of a liquid crystalline phase, the liquid crystalline order discussed above may be examined by use of small-angle X-ray diffraction (SAX), cryo-Transmission Electron Microscopy (cryo-TEM) or Nuclear Magnetic Resonance (NMR) spectroscopy studies. The sizes and size distributions of the dispersed particles may be examined by light scattering or diffraction, particularly by use of laser light diffraction instruments.

Emulsion, micellar or non-lamellar phase formulations are generally formed from mixtures containing at least one amphiphile with at least one hydrophilic "head" group and at least one hydrophobic "tail" group. In most naturally occurring lipids, these groups are joined by an ester linkage with the "tail" consisting of the hydrocarbon chain of the fatty acid(s). Typically, therefore, amphiphile-containing preparations of active agents contain either natural ester-containing lipids, synthetic (i.e. not found in natural extracts) amphiphiles or mixtures thereof.

One disadvantage of the use of ester-containing natural lipids is that the body of a mammalian subject is highly effective in breaking down and absorbing such lipids. This typically occurs by naturally occurring (e.g. digestive) enzymes in the gut or within the body which cleave the relatively labile ester bond, usually to leave a hydrophilic alcohol and a fatty acid or a salt or ion thereof. This lipid degradation can limit the length of time between administration and release of any active agents included in a composition formulated from ester lipids. It also imposes a maximum limit on the period over which an active component may be gradually released. It is therefore desirable to provide a method by which the release profile of a composition, or an active agent formulated therein, can be further controlled and particularly extended while maintaining desirable properties such active agent solubility which may be provided by non-lamellar formulations.

Synthetic, non-natural amphiphiles (i.e. amphiphiles not found in natural product extracts) may be used to reduce the enzymic degradation of amphiphile compositions. The effects and possible degradation products of such non-natural amphiphiles are often unknown, however and therefore it is preferable to avoid having a greater proportion of such components than necessary. It is also not always possible to form stable non-lamellar phases and/or dispersions thereof with such amphiphiles at appropriate temperatures.

The present inventors have now unexpectedly established that by including at least one ether-linked lipid in an amphiphile composition having or forming a non-lamellar phase, the rate of enzymic degradation of the amphiphile may be reduced and the release rate of an active substance formulated therewith further controlled.

In a first aspect, the present invention therefore provides a claim 1. Optionally, the compositions may also contain at least one fatty acid or fatty acid salt, and/or at least one fragmentation agent. The compositions may be bulk phase or preferably a particulate dispersion and will preferably contain at least one aqueous component.

The compositions of the invention will typically be pharmaceutical, cosmetic, dietary or nutritional compositions. Preferably, the compositions will be suitable for administration orally, topically (e.g. to the skin or eye), nasally, parenterally (e.g. subcutaneously or intravenously), rectally or by inhalation. Most preferred are orally administrable compositions such as pharmaceutical, diagnostic, nutritional or veterinary compositions.

In a second aspect, the present invention also provides a method for the production of a particulate composition (preferably of colloidal particles) comprising non-lamellar particles, or forming non-lamellar particles on contact with an aqueous (especially body) fluid, said method comprising forming a mixture comprising an active agent and at least one amphiphile, wherein at least a portion of said amphiphile is an ether-linked lipid, and dispersing said mixture in an aqueous medium.

The method of the invention will typically form compositions suitable for pharmaceutical, cosmetic, dietary or nutritional use. Where particulate compositions are generated, the method may additionally comprise drying the resulting dispersion (e.g. by freeze drying or spray drying) to reduce the proportion of water therein. Such a drying step will typically convert a non-lamellar particulate composition into a composition forming non-lamellar particles on contact with a body fluid.

The components of the compositions include at least one amphiphile of which at least a portion will be an ether-lipid. Where the composition is a particulate composition, they will generally also include a fragmentation agent (which may also be an amphiphile, such as a surfactant, copolymer and/or protein). In addition, the compositions of the invention include an active agent such as a protein, drug, nutrient, cosmetic, diagnostic, pharmaceutical, vitamin, or dietary agents at a level sufficient to be effective. These are referred to herein as "active agents". Under some circumstances the amphiphile and/or fragmentation agent may also be an active agent, although it is preferred that the active agent should not be an ether lipid, a structure forming amphiphile or a fragmentation agent.

It is preferable that the thermodynamic equilibrium state of the component mixture of the formulation, optionally in the presence of excess solvent (such as water) is a non-lamellar phase (such as the normal or reversed cubic or hexagonal phases or L₃ phase) or L₂ phse. Where a non-lamellar phase is the thermodynamic or near-thermodynamic state of a formulation then (especially where the mixture includes a fragmentation agent) particles of non-lamellar phase may form, for example from an L₂ or homogeneous oil phase, on contact with aqueous media, either *in vitro* or *in vivo*. Where such formulations release or generate fragments of non-lamellar phase *in vivo* they are forming such non-lamellar phase particles on contact with a body fluid and are thus examples of non-lamellar phase forming compositions of the invention. Such non-lamellar phase precursors form a preferred aspect of the invention and may contain any of the additional components of the non-lamellar compositions or formulations of the invention (e.g. fragmentation agents and excipients).

Typically, the non-lamellar precursor compositions of the invention will contain a lower proportion of aqueous component than the non-lamellar compositions themselves but will contain other components (especially amphiphilic components, active agents and fragmentation agents) in equivalent relative proportions to the non-lamellar particles they will form. Non-lamellar precursor compositions may contain a larger relative proportion of aqueous soluble materials such as buffers, salts and sugars. These will be included in the composition precursors so as to control the properties of the aqueous component formed upon exposure to an aqueous medium (particularly a body fluid). They may also serve to protect the particulates during drying and/or manipulation.

The non-lamellar precursor compositions will typically be a bulk lipid phase or a reversed micellar (e.g. L₂ phase) particulate composition.

As use herein, the term "non-lamellar" is used to indicate a normal or reversed liquid crystalline phase (such as a cubic or hexagonal phase) or the L₃ phase or any combination thereof. Where a particle is described as having a non- lamellar phase or form, this indicates that amphiphiles in at least the internal region of the particle should adopt this form. The particles will generally have two distinct regions, an internal region and a surrounding surface region. The surface region, even in a "non-lamellar" particle will typically be lamellar, L₃ or crystalline. In contrast, a "lamellar" particle, as described herein is a particle having a solvent, rather than non-lamellar, core-region. Preferred non-lamellar forms are reversed liquid crystalline phases such as cubic or hexagonal phases. Most preferred is the reversed hexagonal phase.

Dispersions containing active ingredients and particularly those for intravenous administration to the human or animal body are desirably colloidal, that is they should be of a particle size no greater than 10 µm, especially no greater than 5 µm and particularly no greater than 1 µm. If particles within the dispersion exceed this size then the dispersion may not be colloidally stable and there is a considerable risk of causing embolism when the preparation is administered intravenously. Furthermore, it is desirable that the distribution of particle sizes be narrow to maximise control over the release of any active agent. Compositions of the invention for intravenous delivery are therefore desirably colloidal as indicated above.

As used herein, the term "particle size" indicates the mean particle size of dry powder or dispersed particles along the longest axis thereof. Generally, a particle size distribution will be considered narrow if it is monomodal and the width at half-height is no greater than the average particle size.

Where a composition is to be administered by a method other than intravenously (e.g. orally, intramuscularly, subcutaneously, rectally or by inhalation), then the particles need not be colloidal. Bulk phases such as liquid crystal phases may be administered orally, for example. It remains, however, advantageous for particulate compositions to provide well characterised and reproducible particle size distributions in order to control the rate of decomposition of the particles and/or release of the active agents.

A composition is considered "non-lamellar" if a molecular fraction of at least 30% of the structure forming amphiphile exists as a bulk non-lamellar phase, as non-lamellar phase particles, or as a combination thereof. Similarly, a composition forms non-lamellar phase or particles if at least 30% of the amphiphile is in the form of such phases after exposure to an aqueous fluid. This will generally be at least 50% in both cases and preferably at least 70% of the amphiphilic component should be in a non-lamellar form, either in the composition as formulated or after exposure to a body fluid. More preferably this is at least 80%, most preferably 90% or more.

The term amphiphile as used herein in the methods and compositions of the invention includes any agents that are capable of forming a structured phase in the presence of an aqueous solvent, optionally in the presence of other agents such as other amphiphiles and/or fragmentation agents. Amphiphiles will have at least one polar, hydrophilic group and at least one non-polar, hydrophobic group.

Examples of polar groups are well known (see e.g. US 2002/0153509) and include anionic groups such as carboxylates, phosphonates, sulphates and sulphonates, non-ionic groups such as alcohols, polyols (e.g. sugars glycerol) and esters, cationic groups such as quaternary ammonium compounds, pyridinium salts and quaternary phosphonium salts and zwitterionic groups such as phospholipid head groups (e.g. phosphatidyl-choline.), ammonioacetates, ammonio-alkanesulphonates and trialkylaminoalkylphosphate esters.

Examples of non-polar groups include C₆-C₃₂ alkyl and alkenyl groups, which are typically present as the esters of long chain carboxylic acids. These are often described by reference to the number of carbon atoms and the number of unsaturations in the carbon chain. Thus, CX:Z indicates a hydrocarbon chain having X carbon atoms and Z unsaturations. Examples particularly include caproyl (C6: 0), capryloyl (C8:0), capryl (C10:0), lauroyl (C12:0), myristoyl (C14:0), palmitoyl (C16:0), phytanolyl (C16:0), palmitoleoyl (C16:1), stearoyl (C18:0), oleoyl (C18:1), elaidoyl (C18:1), linoleoyl (C18:2), linolenoyl (C18:3), arachidonoyl (C20:4), behenoyl (C22:0) and lignoceroyl (C24:9) groups. An amphiphile will typically have one or two non-polar "tail" groups (mono-acyl and di-acyl lipids respectively in the case of ester lipids or mono-alkyl and di-alkyl respectively in the case of ether lipids) but may have three, four or more hydrophobic groups.

Examples of amphiphiles suitable for use in the present invention include natural lipids, synthetic lipids, surfactants, copolymers, proteins (in particular caseins and albumin), hydrotropes, alcohols, and other additives that may form or facilitate formation of structured phases. Preferred agents are glycerides (e.g. monoglycerides, diglycerides, and triglycerides), di- and polyglycerolesters of glycerides (e.g. diglycerol monooleate, diglycerol monocaprate), natural fats and oils (e.g. soybean oil, coconut oil, corn oil, castor oil, sunflower oil), fractionated oils (e.g. fractionated coconut oil, Miglyol® (Condea)), transesterified oils (e.g. Maizine®), transesterification products of oils and PEG (e.g. ethoxylated castor oil (e.g. Cremophor® EL (BASF)), ethoxylated hydrogenated castor oil (e.g. Cremophor® RH-40 (BASF)), ethoxylated corn oil (e.g. Labrafil® M 2125 CS (Gattefossé))), acetylated monoglycerides, fatty acids (e.g. C6-C26 saturated and unsaturated fatty acids), fatty alcohols (e.g. phytantriol (3,7,11,15-tetramethyl-1,2,3-hexadecantriol)), ether lipids (e.g. monooleyl glyceryl ether, glyceryl monophytanyl, phytanyl glycolipids, and others indicated below), natural and synthetic phospholipids (e.g. egg lecithin, soya lecithin, hydroxylated lecithin, phosphatidyl choline, phosphatidyl ethanolamine, phosphatidyl serine, phosphatidyl glycerol, phosphatidic acid), lysophospholipids (e.g. lyso-lecithin, lyso-phosphatidyl choline, lyso-oleyl phosphatidyl choline), phospholipid-analogous compounds (e.g. those disclosed in US 6344576), sterols and sterol derivatives (e.g. cholesterol, sitosterol, lanesterol and their esters, especially with PEG or fatty acids), galactolipids (e.g. digalactosyl diacylglycerol, monogalactosyl diacylglycerol), sphingolipids (e.g. sphingomyelin); nonionic surfactants, in particular ethoxylated surfactants such as PEG-fatty acid mono- and diesters (e.g. of the Crodet® (Croda), Cithrol® (Croda), Nikkol® (Nikko), Myrj® (ICI) series, Solutol® HS 15 (BASF)), PEG glycerol fatty acid esters (e.g. Tagat® L and O (Goldschmidt), Glycerox® L series (Croda), Capmul® EMG (Abitec)), transesterification products of oils and PEG (e.g. of the Labra fil® (Gattefossé), Cremophor® (BASF) Crovol® (Croda) and Nikkol® HCO (Nikko) series), PEG-sorbitan fatty acid esters (e.g. Tween® 20, Tween® 80 and other polysorbates of the Tween® series (ICI)), PEG alkyl esters (e.g. of the Brij® (ICI) and Volpo® (Croda) series), PEG alkyl phenol surfactants (e.g. of the Triton X and N series (Rohm & Haas); polyglycerised fatty acids (e.g. Nikkol® Decaglyn (Nikko), Plurol® Oleique (Gattefossé)), propylene glycol fatty acid esters), propylene glycol fatty acid esters (e.g. Capryol® 90 (Gattefossé), Lutrol® OP2000 (BASF), Captex® (Abitec)), glycerol/propylene glycol fatty acid esters (e.g. Arlacel® 186 (ICI)), sorbitan fatty acid esters (e.g. of the Span® (ICI) and Crill® (Croda) series), sugar esters (e.g. of the SUCRO ESTER® (Gattefossé) and Crodesta® (Croda) series), polyoxyethylene-polyoxypropylene block copolymers (so-called poloxamers, e.g. of the Pluronic® (BASF), Synperonic® (ICI) and Lutrol® (BASF) series), copolymers of ethylene oxide and butylene oxide; anionic surfactants including fatty acid salts, bile salts (e.g. sodium cholate, sodium glycocholate, sodium taurocholate), carboxylates such as ether carboxylates, succinylated monoglycerides, mono/diacetylated tartaric acid esters of mono- and diglycerides, citric acid esters of mono- and diglycerides, glyceryl-lacto esters of fatty acids, acyl lactylates, alginate salts, propylene glycol alginate; cationic surfactants including ethoxylated amines (e.g. polyoxyethylene-15 coconut amine), betaines (e.g. N-lauryl-N,N-dimethylglycine), alkylpyridinium salts, quarternary ammonium salts such as hexadecyl triammonium bromide, decyl trimethyl ammonium bromide, cetyl trimethyl ammonium bromide; zwitterionic surfactants including trimethylammonioethylalkylphosphonates (e.g. the examples disclosed in US 6344576); and all mixtures thereof. The most preferred structuring agents for inclusion with ether-lipids indicated below are glyceryl monooleate, glyceryl monolinoleate, glyceryl dioleate, di-glycerol monooleate, dioleyl phosphatidyl ethanolamine (DOPE), dioleyl phosphatidylcholine (DOPC) and phytantriol, and mixtures of these with up to 50% fatty acids, in particular oleic acid and linoleic acid, polysorbate 80 (Tween® 80), polyethylene glycol 660 12-hydroxysterate (Solutol® HS 15), or lyso-phospholipids, especially lyso-oleyl phosphatidylcholine (LOPC). Naturally occuring lipids from natural or synthetic sources are preferred due to their generally lower and/or more predictable toxicity profile.

Often the amphiphilic component will contain material in the form of extracted and purified natural products and will thus contain a mixture of related compounds. Soy bean phosphatidyl choline, for example is a mixture of compounds having around 60-75% C18:2 acyl groups, around 12-16% C16:0 and the balance others.

Similarly, commercial glyceryl monooleate is typically at least 90% monoglyceride with the acyl groups being over 60-90% C18:1, 5-10% saturated and the remainder largely higher unsaturated acyl groups, but containing small amounts of diglycerides and free fatty acids. Different commercial preparations will also vary slightly.

A highly preferred structuring agent for use in the present invention is commercially available glyceryl monooleate (GMO). As indicated above, this is largely monoglyceride with an oleoyl (C18:1) acyl chain but contains certain amounts of other compounds. These are included in the term "glyceryl monooleate" or "GMO" as used herein. Commercial preparations of GMO include GMOrphic-80 and Myverol 18-99 (available from Eastman Kodak), Rylo MG 19 and Dimodan DGMO (available from Danisco). Any of the structure-forming amphiphiles may be used alone or in combination with one or more other amphiphilic structuring agents.

The key amphiphilic component of the compositions, composition precursors and methods of the present invention are referred to herein as "ether lipids" or "ether linked lipids". As used herein, these are amphiphiles having a polar group attached to at least one non-polar "tail" by an ether linkage.

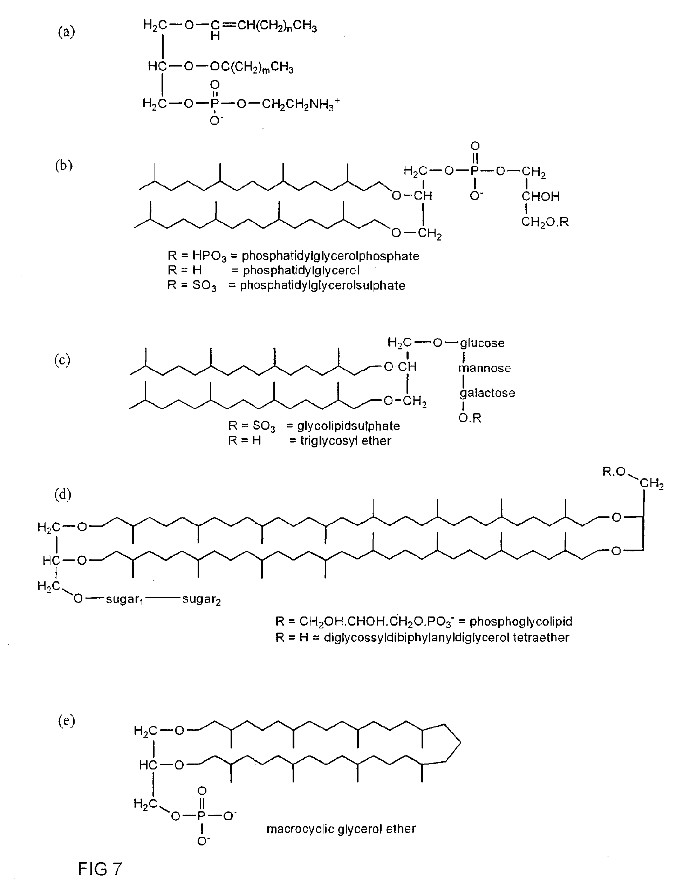

Many ether lipids are natural products occurring, for example, in both human and cows' milk. Glycerol ethers, for example, are particularly abundant in oily extracts from certain marine creatures, especially the livers of cold water sharks. Some ether lipids are also present in the mammalian gut during digestion of certain foods. Ether lipids are also present in the cell walls of certain bacteria. Anerobic bacteria, including those of the rumen contain quite large amounts of plasmalogens (see (a) in Figure 7). Extreme halophiles contain large amounts of phosphatidylglycerol-sulphate, diphytanylglycerol diethers and dibiphytanyldiglycerol tetraethers ((b) to (d) respectively in Figure 7) and certain thermophilic bacteria contain macrocyclic diethers ((e) in Figure 7).

Figure 7 shows the structures of bacterial ether lipids: (a) anaerobic bacterial plasmaogens; (b)-(e) archaebacterial phytanyl ethers. In (d), the sugars are usually glucose and galactose.

The standard process for the production of monoacylglycerols (known as "mollecular distillation" in the art) can be directly applied to either lipid production by replacing the fatty acid by a fatty alcohol. Monoalkly ether lipids can also be produced on an industrial scale by hydrolysis of natural ether containing lipids, especially from marine sources.

One preferred method for the synthesis of ether lipids is to react the salt (e.g. alcohol potassium salt) of a protected hydrophilic group with an activated fatty alcohol (e.g. a tosylated fatty alcohol). Removal of the protecting group then leaves an ether lipid.

For 1-alkyl glycerols, a general synthesis from the acetal protected 1,2-isopropylidene glycerol and p-toluene sulphonate protected fatty alcohol is indicated below:

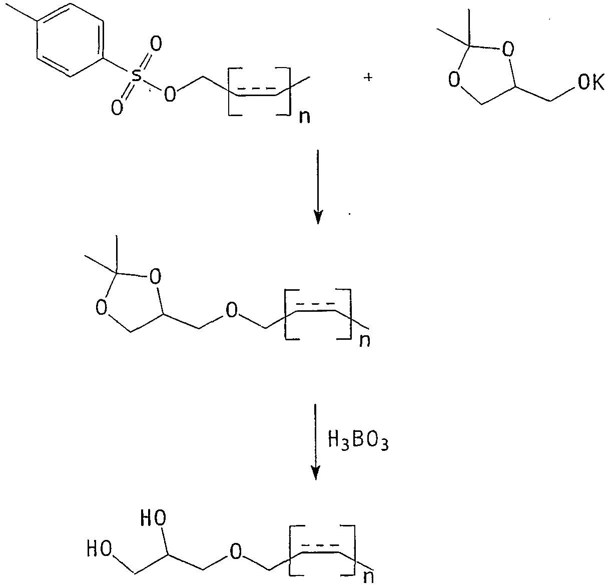

The above synthetic method may easily be adapted to provide mono- or di-acyl (e.g.acetyl) alkylglycerols by subsequent optional protection and esterification with fatty acids. Similarly, other polyols (e.g. ethylene glycol) may be suitably protected (e.g. as benzyl ethers or acetals) and etherified by the above method.

Preferred polar "head" groups in the ether lipids for use in the present invention include glycerol, choline, ethanolamine, their esterified (e.g. by succinic acid), glycosylated and/or phosphated equivalents and mixtures thereof. The most preferred polar group is glycerol.

Polar groups in ether lipids may be attached to one, two, three or more non -polar tail groups of which at least one will be attached by an ether linkage. Where more than one non-polar group is present, only one need be attached by an ether group and the remainder may be attached by other linkages. Typically one ether bonded non-polar group will be present and any other tail groups will be attached ester bonds. In the case of glycerol, the most preferred configuration is for a first non-polar group to be attached by an ether group at the 1- position with optional additional non-polar groups attached by ester groups at the 2- and/or 3- positions. Most preferably, the 2- and 3-positions are unsubstituted. In a particularly preferred embodiment the ether lipid does not contain an ester group.

Preferred ether lipids are mono-, di- and tri-glyceride analogues where one or more fatty acid (acyl) residues is replaced by a hydrocarbyl residue, where these residues are optionally unsaturated groups with 6 to 24 carbon atoms and are preferably linear. These may be conveniently prepared by the method indicated above or from the hydrolysis of natural 1-alkyl-2,3-diacyl glycerols.

Alternatively, the ether lipids may be mono- or di-alkyl substituted glycols and for 1-alkyl glycols the, 2-position may be substituted with an alcohol. These alkyl glycols may be synthesised by simple modification of the process indicated above.

Preferred non-polar tail groups will be fatty alcohols where they are ether-linked and will generally be linear with 6 to 24 carbon atoms. These groups will have the formula -O-CₓH_{y} where the hydrocarbon portion is saturated (y=2x+1) or has one or more unsaturations (y=2x+1-2z where z is the number of unsaturations). These groups are typically represented by the notation Cx:z where x is the number of carbons and z is the number of unsaturations. For example, C14:1 represents a carbon chain having 14 carbons and 1 unsaturation, which is thus an -O-C₁₄H₂₇ fatty alcohol. Preferred non-polar groups are alcohols and acids represented as C6:0, C8:0, C10:0, C12:0, C13:0, C14:0, C16:0, C16:1 (especially 9-cis or 9-trans), C18:0, C18:1 (especially 6-cis, 9-cis or 9-trans), C18:2 (especially 9-cis-12-cis), C18:3 (especially 9-cis-12-cis-15-cis), C20:4 (especially 5, 8, 11, 14 (all cis)), C22:0, C22:1 (especially 13-cis), C22:6 (especially 4, 7, 10, 13, 16, 19 (all cis)), C24:1 (especially 15-cis) and C24:9. This includes branched-chain fatty acids and alcohols but the straight chain groups are preferred.

The most preferred ether lipids comprise predominantly glycerol polar groups with a C18:1 (especially straight chain 6-cis, 9-cis or 9-trans) fatty alcohol group attached by a linking ether group at the 1-position. The ether lipid may also optionally have other non-polar groups attached to the 2- and/or 3- positions by ester linkages but is preferably unsubstituted in these positions. Most preferred either lipid is glyceryl-1-oleyl ether (GEO).

The ether lipids used in the present invention typically occur naturally in marine creatures and so may be present in certain foods. When typical ether containing lipid such as 1-alkyl-2,3-diacyl-glycerol is digested in the gut, 1-alkyl-glycerol is thought to be formed. Such ether lipids are therefore expected to be very well tolerated by a subject. Certain ether lipids may also, in themselves, have a beneficial effect upon the subject, for example by having antibiotic properties or by boosting the mammalian immune system. Although this is a less preferred embodiment, the ether lipids may in some circumstances form an active agent in the composition. This is especially for the treatment or prophylaxis of cancers, in immune modulation or in systemic or topical treatment of certain skin conditions such as psoriasis. It is preferable that where the ether lipid is present as an active agent, an additional active agent not being an ether lipid, a structure forming amphiphile or a fragmentation agent is also formulated in the composition.

Although biologically tolerable, the ether lipids present in the compositions of the invention are degraded less quickly after administration than more common natural lipids containing only ester linkages between the polar and non-polar group(s). The ether linkage is stable to the action of esterases which typically degrade ester bonds *in vivo* and can furthermore act as inhibitors of these enzymes. The lifetime of the compositions of the present invention may therefore be considerably increased relative to equivalent compositions made with only ester-linked lipids. In a preferred embodiment, the present invention therefore provides a controlled release formulation comprising a composition of the invention.

Control over the rate of degradation may also be exercised by using a mixture comprising part ester lipids and part ether lipids, thereby forming a composition with a controlled intermediate rate of disintegration. Equally, for a particulate composition, two or more separate dispersions may be prepared, for example one with ester lipids and the other with ether lipids, and the dispersions mixed. This would give a final composition containing particles with at least two different release profiles and might be desirable, for example, where rapid effectiveness was required in combination with a long period of sustained drug release. In a further embodiment of the invention, therefore, the compositions of the invention may comprise part ether lipid and part ester lipid. Where the composition is a particulate composition, it may comprise a mixture of particles having different proportions of ether lipid providing that, taken as a whole, the composition is a composition of the invention as described herein.

By appropriate selection of the type and proportion of active agent, amphiphile, fragmentation agent, fatty acid or fatty acid salt and ether lipid as appropriate, control may be exercised over the phase, size, physical stability and chemical (e.g. enzyme degradative) stability of the particles in dispersed compositions of the invention. Smaller size particles will typically give more rapid release of active agent due to larger surface areas. Larger proportions of ether lipid will tend to give less rapid release due to slower degradation of the ether lipid forming the carrier. Certain methods of delivery demand that some of these parameters be fixed (e.g. the dispersion may need to be colloidal for delivery by intravenous injection) but by using appropriate control over the available variables the skilled worker will be able to provide compositions with the most desirable release profile.

The compositions of the invention will generally comprise sufficient ether lipid to provide the desired rate of degradation of the composition. This will generally be at least 5% by weight ether lipid (relative to the total amphiphile content, including any fragmentation agent) but will more preferably be at least 20%, for example at least 40% by weight. Preferably, the ether lipid will be present as at least 50 wt% of the amphiphilic component and this may be at least 75%, at least 90% or at least 95%. In one preferred embodiment, essentially all of the amphiphilic component consists of ether lipid and any optional fragmentation agent.

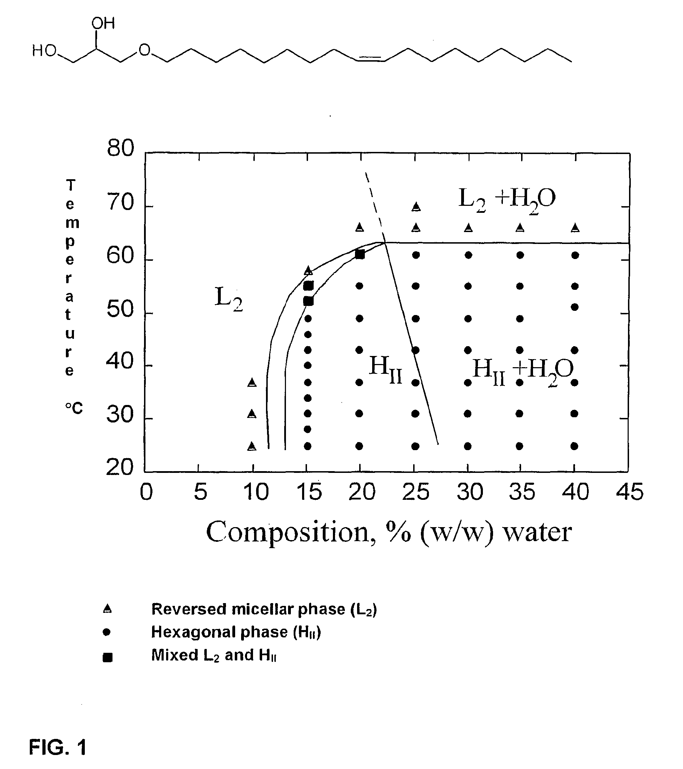

The ether lipids present in the compositions of the invention can form bulk or dispersed liquid crystal phases both in essentially pure form and as mixtures with ester lipids. A phase diagram indicating the phases formed by mixtures of the ether lipid 1-(cis-octadec-9-ene) substituted glycerol with water is shown in Figure 1. This phase diagram indicates that the ether lipid forms a reversed hexagonal (H_{II}) phase at water concentrations of above around 15% and at higher concentrations this liquid crystalline form exists stably with excess water. At lower water concentrations the reversed micellar (L₂) phase is formed. The micelles of the L₂ phase for this ether lipid are an example of a composition which will form a non-lamellar phase upon contact with a body fluid, since as the water content increases the composition will enter the H_{II} region and thus form stable hexagonal liquid crystal particles. The exemplified ether lipid thus forms valuable structures over a considerable range of water concentrations at both room temperature and physiological temperature.

A further demonstration of a composition with properties equivalent to those of the compositions of the invention (in the absence of an active agent) is given in Example 6 below. In this Example, compositions of ether lipid with poloxamer and optionally a fatty acid salt and/or ester lipid are prepared. These compositions form non-lamellar phases by dispersion in contact with aqueous solutions, as indicated below. In the presence of at least one active agent, these would therefore be compositions of the invention, as would be the non-lamellar dispersions.

In compositions of the invention which form non-lamellar phases on contact with a body fluid, the body fluid will depend upon the mode of administration. Typically, for example, oral compositions will form non-lamellar phases upon contact with gastric or intestinal fluid, parenteral compositions will form such phases on contact with blood and inhalable compositions will form non-lamellar phases on contact with fluids in the lungs and bronchial passages. In the case of topical compositions, these will preferably be of non-lamellar phase prior to application but may also form such phases on contact with fluids such as sweat or fluid on the surface of the eye.

In addition to the amphiphilic component, the compositions of the invention may include at least one fatty acid or fatty acid salt component. Preferred fatty acids are those corresponding to the fatty acid chains of natural ester lipids, including caproic, caprylic, capric, lauric, myristic, palmitic, phytanic, palmitolic, stearic, oleic, elaidic, linoleic, linolenic, arachidonic, behenic or lignoceric acids, their salts or mixtures thereof. Salts of fatty acids will typically be physiologically tolerable, and for pharmaceutical applications will always be so. Preferred salts include alkali and alkaline earth metal salts such as sodium, potassium, lithium, calcium or magnesium salts as well as ammonium and alkylammonium salts.

The compositions of the invention will typically include at least one fragmentation agent, particularly where the composition is a dispersion. Suitable fragmentation agents will be agents which aid the dispersal of amphiphile into particles (especially non-lamellar phase particles) or stabilise such particles. Typically a fragmentation agent will be a surfactant such as an amphiphilic block copolymer.

Important fragmentation agents include natural lipids, synthetic lipids, surfactants, copolymers, proteins (in particular caseins and albumin), hydrotropes, alcohols and other additives that may facilitate fragmentation spontaneously or with the aid of externally applied forces and pressures and contribute to stabilisation. This includes also nanoparticles and combinations of polymer and nanoparticles (see e.g. WO 99/12640).

Preferred fragmentation agents are surfactants, polymers and copolymers and these copolymers may have blocks comprising polyoxyalkylenes, polyvinylpyrollidone, polyvinylacetate, polyvinylalcohol, polyesters, polyamides and/or polyalkenes. The block copolymer will comprise at least two blocks of polymer having different degrees of hydrophillicity. Certain proteins (such as casein) are also of amphiphilic character and may be used as fragmentation agents. Where an active agent is an amphiphilic protein, this may act as both the active agent and the fragmentation agent, or may be included in addition to another active agent and/or fragmentation agent. The fragmentation agent will generally not be an ether lipid.

Preferred examples of amphiphilic block copolymers are poloxamers, which comprise at least one block of polyoxyethylene and block of polyoxypropylene. The most preferred fragmentation agents are poloxamer 407 (e.g. Pluronic® (Lutrol) F127, BASF), poloxamer 188 (e.g. Pluronic® F68, BASF), poloxamer 124 (Pluronic® L44, BASF), and polysorbates 20, 60 and/or 80 (referred to herein a P20, P60 & P80 respectively - e.g. Tween® 80, ICI)

Where included to aid dispersion, the fragmentation agent will be present at a level sufficient to bring about the fragmentation of the composition and/or to stabilise the fragmented particles (which will preferably be non-lamellar phase). Such fragmentation may be spontaneous or may require physical fragmentation such as by sheering and/or ultrasonication. It is preferable that sufficient fragmentation agent is present that the composition is physically stable. Typically a fragmentation will provide a desired effect at a level of 1-50% by weight, relative to the total amphiphile content of the composition. This will more typically be 2-15% by weight.

Active agents suitable for inclusion in the methods and formulations of the present invention include human and veterinary drugs and vaccines, diagnostic agents, cosmetic agents, nutrients, dietary supplements etc. Examples of suitable drugs include antibacterial agents such as β-lactams or macrocyclic peptide antibiotics, anti fungal agents such as polyene macrolides (e.g amphotericin B) or azole antifungals, anticancer and/or anti viral drugs such as nucleoside analogues, paclitaxel and derivatives thereof, anti inflammatorys, such as non-steroidal anti inflammatory drugs, cardiovascular drugs including cholesterol lowering and blood-pressure lowing agents, analgesics, antidepressants including seritonin uptake inhibitors, vaccines and bone modulators. Diagnostic agents include radionuclide labelled compounds and contrast agents including X-ray, ultrasound and MRI contrast enhancing agents. Nutrients include vitamins, coenzymes, dietary supplements etc. The active agents for use in the present invention will generally not be poloxamers, fragmentation agents, structure forming amphiphiles or ether lipids.

Preferred active agents include human and veterinary drugs selected from the group consisting of peptides such as adrenocorticotropic hormone (ACTH) and its fragments, angiotensin and its related peptides, antibodies and their fragments, antigens and their fragments, atrial natriuretic peptides, bioadhesive peptides, Bradykinins and their related peptides, calcitonins and their related peptides, cell surface receptor protein fragments, chemotactic peptides, cyclosporins, cytokines, Dynorphins and their related peptides, endorphins and P-lidotropin fragments, enkephalin and their related proteins, enzyme inhibitors, fibronectin fragments and their related peptides, gastrointestinal peptides, growth hormone releasing peptides, immunostimulating peptides, insulins and insulin-like growth factors, interleukins, luthenizing hormone releasing hormones (LHRH) and their related peptides, melanocyte stimulating hormones and their related peptides, nuclear localization signal related peptides, neurotensins and their related peptides, neurotransmitter peptides, opioid peptides, oxytocins, vasopressins and their related peptides, parathyroid hormone and its fragments, protein kinases and their related peptides, somatostatins and their related peptides, substance P and its related peptides, transforming growth factors (TGF) and their related peptides, tumor necrosis factor fragments, toxins and toxoids and functional peptides such as anticancer peptides including angiostatins, antihypertension peptides, anti-blood clotting peptides, and antimicrobial peptides; selected from the group consisting of proteins such as immunoglobulins, angiogenins, bone morphogenic proteins, chemokines, colony stimulating factors (CSF), cytokines, growth factors, interferons, interleukins, leptins, leukemia inhibitory factors, stem cell factors, transforming growth factors and tumor necrosis factors; selected from the group consisting of antivirals, steroidal antiinflammatory drugs (SAID), non-steroidal anti-inflammatory drugs (NSAID), antibiotics, antifungals, antivirals, vitamins, hormones, retinoic acid, prostaglandins, prostacyclins, anticancer drugs, antimetabolic drugs, miotics, cholinergics, adrenergic antagonists, anticonvulsants, antianxiety agents, tranquilizers, antidepressants, anesthetics, analgesics, anabolic steroids, estrogens, progesterones, glycosaminoglycans, polynucleotides, immunosuppressants and immunostimulants, cardiovascular drugs including lipid lowering agents and blood-pressure lowering agents, bone modulators; vaccines, vaccine adjuvants, immunoglobulins and antisera; diagnostic agents; cosmetic agents, sunscreens and self-tanning agents; nutrients; dietary supplements; herbicides, pesticides, and repellents. Further examples of active agents can be found for instance in Martindale, The Extra Pharmacopoeia. Particularly preferred active agents include enzyme susceptible active agents such as peptides and agents of poor solubility such as cyclosporins.

Where an active agent is amphiphilic or of low solubility in water and oil, it is preferred that the compositions of the invention incorporating that active agent are dispersions of non-lamellar phase, such as reversed cubic or hexagonal structures.

In the methods of the invention, a mixture is formed comprising at least one amphiphile comprising an ether lipid, at least one active agent, optionally an aqueous component and optionally at least one fragmentation agent. This composition precursor is then dispersed in a solvent (typically an aqueous solvent) to provide a particulate composition. Preferably, at least a portion of the particles of such a dispersed composition will be non-lamellar particles. The particles may be at least partially micellar (L₂) phase particles or less desirably they may be at least partially lamellar particles such as liposomes, providing that non-lamellar phase particles are formed on exposure to body fluids.

In an alternative method of the invention, an amphiphile comprising an ether lipid may be formed into a dispersion in the absence of active agent and the active agent introduced by, for example, incubating the particles of the dispersion in a solution (especially aqueous solution) of the active agent. This method is most suitable where the active agent is at least partially water soluble and/or where the dispersion process or a subsequent step (such as heat treatment as below) might cause the breakdown of the active agent.

The composition precursors and dispersions may be formed by established methods using ether lipid as a portion of the amphiphilic component. Suitable methods include those indicated in the present Examples and in US 5,531,925, WO 02/02716, WO 02/068561, WO 02/066014 and WO 02/068562.

Dispersion methods include adding an amphiphile/water liquid crystal phase to an aqueous solution of fragmentation agent and optionally a lipid (such as phosphatidyl choline - PC) and either allowing natural fragmentation of the mixture or accelerating the process with, for example, mechanical agitation, vortexing, roto - stator mixing, high-pressure homogenation, microfluidisation and/or ultrasound.

The phase behaviour and size distribution of particulate formulations of the invention may also be controlled by one or more (preferably one) cycles of heating and cooling. Such cycles can be used to convert lamellar particles to non-lamellar form, and to reduce the spread of particle sizes. Where this method is used, the compositions should, preferably, be formulated such that the thermodynamically stable state is non-lamellar. Where heat cycling is used, the active agent may be incorporated into the particles prior to and/or after heat cycling. Where more than one heat cycle is used, the active agent may also or alternatively be incorporated between cycles. Where the active agent is heat sensitive (e.g. peptide or protein) the active agent is preferably incorporated only after heat cycling is complete.

A heat cycle brings the composition, with or without the active agent present, up to a temperature sufficient to provide conversion of at least a portion of the particles to non-lamellar phase upon cooling to ambient temperature. This will typically involve heating to around 90-150°C for 1-30 min followed by cooling to ambient temperature. More typically a heat cycle will involve heating to 100-130°C (e.g. 100-120°C) for 2-20 minutes before cooling. The most suitable conditions will vary in detail between compositions but will be readily established by the skilled worker.

In the heat cycling process, the mean particle size typically increases but the distribution of particle sizes can be reduced.

In one preferred embodiment, at least one heat cycle as described above may be used to enhance the loading of a composition of the invention with active agent. In this method, a preferably sparingly soluble active agent is mixed with an aqueous suspension of particles comprising amphiphiles as described herein and the suspension heated, followed by cooling as described above. The present inventors have unexpectedly noted that this method can provide a much higher stable loading of active agent than is possible by equilibration at room temperature.

The presence of particles in non-lamellar form will preferably be assessed from a set of cryo-transmission electron microscopy particle images, preferably showing a sample of more than 30, preferably more than 50, more preferably more than 100 particles. The presence of non-lamellar particles may also be assessed by X-ray scattering experiments.

The compositions of the invention may be used in the production of nutritional, dietary, cosmetic, diagnostic or pharmaceutical products by known methods using well known carriers, excipients and other ingredients. In the case of pharmaceutical compositions, the particles will be formulated with at least one pharmaceutically acceptable carrier or excipient and may be formed into tablets, capsules and so forth. Particulate compositions may also be formulated as a pre-prepared dispersion in an acceptable liquid, such as water, or dried and sealed in sterile containers for resuspension prior to administration.

Preferred formulations for pharmaceutical administration include topical formulations such as creams, gels, eye-drops or aerosols; oral compositions such as dispersions, powders, powder or liquid filled capsules, tablets, coated tablets or coated capsules; parenteral formulations such as dispersions in buffers, saline; rectal formulations such as suppositories; and inhalable formulations such as aerosols or aerodynamic powders. Suitable carriers and excipients will be known to one of ordinary skill and include purified water, osmolality adjusters such as salts, pH buffers, pH adjusters, cryoprotectants such as sugars, soluble polymers such as polyethylene glycol or polyvinylpyrrolydone, flavours, sweeteners and colours fillers.

Formulations for cosmetic purposes will typically be topical formulations such as creams, gels, aerosols, waxy sticks, foams, mousses or liquids such as dispersions for roll-on applications or for filling liquid-pens. Suitable carriers and excipients will again be well known and include liquids such as water and alcohols (such as ethanol or propanol); waxes, colourants, fillers, perfumes, opacity increasing agents such as titanium dioxide and gelling agents.

Topical creams and gels and oral formulations are the preferred formulations of the compositions of the invention. Oral formulations are most preferred.

The present invention is further described below by reference to the Examples and to the attached Figures, in which:
Figure 1 represents the phase diagram of mixtures of GEO and water over a range of temperatures;
Figure 2 shows the equilibrium phase of GEO with 5% CsA in water;
Figure 3 shows the equilibrium phase of GEO in 10% CsA;
Figure 4 shows the equilibrium phase of GEO/GMO (10/9) in water;
Figure 5 shows the equilibrium phase of GEO/GMO (10/9) with 5% CsA;
Figure 6 shows the equilibrium phase of GEO/GMO (10/9) with 10% CsA;
Figure 7 shows several bacterial ether lipids;
Figure 8a shows the results of a GMO/GEO/water phase study;
Figure 8b shows a triangular GMO/GEO/water phase diagram;
Figue 9a shows the pseudo-two-dimensional plane of GMO/GEO/water having 10% F 127 to total amphiphile;
Figure 9b shows the equilibrium phases formed in the GMO/GEO/F127/water system;
Figure 10 shows the particle size distributions of GMO/F 127 and GMO/GEO/F127 systems; and
Figure 11 shows the effect of lipase treatment in generating free acid by degradation of the GMO/F127 and GMO/GEO/F127 systems.

### Example 1 - Preparation of GEO

Glyceryl-1-monooleate ether (GEO, also abbreviated to GME) can be obtained commercially from Nikkol® Japan in 95% purity. GEO of greater purity was synthesised by the reaction of 1,2-isopropylidene glycerol potassium salt and oleyl p-toluene sulphonate as indicated in the reaction scheme below. The progress of the reaction was followed by TLC.

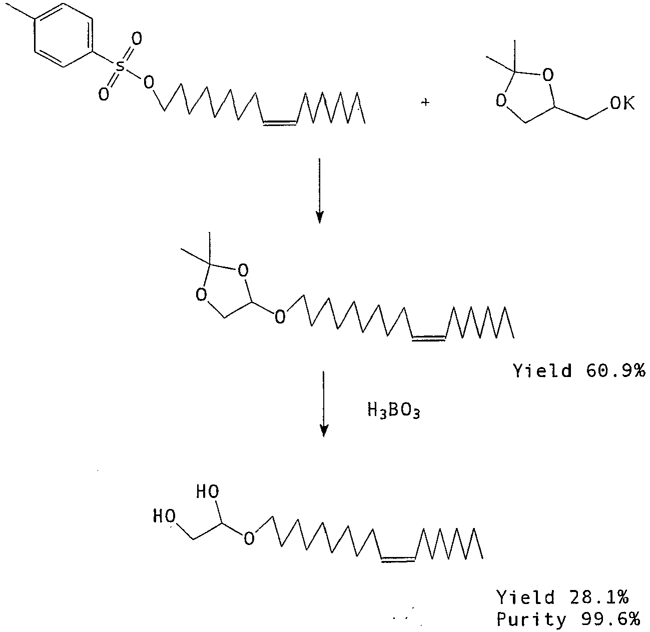

### Example 2 - Phase diagram for GEO

Mixtures of glyceryl-1-monooleate ether (GEO) with water were prepared, shaken and allowed to equilibrate at a known temperature. The phase(s) of the resulting mixture were examined under the polarising microscope and by small angle x-ray diffraction.

The results are indicated in Figure 1, wherein triangles indicate the presence of L₂ phase, circles indicate the presence of reversed hexagonal (H_{II}) phase and squares indicated the co-existence of both L₂ and H_{II} phases. At higher water content, both the L₂ and the H_{II} phases were stable in the presence of excess water.

The experiment indicates that stable reversed micellar and reversed hexagonal phases exist in the phase diagram of GEO/water at temperatures spanning room temperature and ambient temperature. As the water content increases, the compositions progress from L₂ phase to pure H_{II} phase to H_{II} phase in equilibrium with excess water, indicating that an L₂ phase composition would be suitable as a non-lamellar phase precursor composition. Such a composition would form hexagonal phase when diluted by the water content of body fluids.

### Example 3 - Solubility of Cyclosporin

Cyclosporin A (CsA) in lipid mixtures was prepared at concentrations indicated in Table 1 below. The mixtures were prepared and allowed to equilibrate for 12 hours at 40 C. Samples were assessed for CsA solubility and then allowed to cool to room temperature before being reassessed. The results are indicated in Table 1, wherein indicates solubility (no CsA crystals were present) and x indicates insolubility (crystals of CsA observed).

**Table 1**

| CsA (wt%) | GEO | | GEO/GMO (10:9) | |
|---|---|---|---|---|
| | 40°C | R.T. | 40°C | R.T. |
| 5% | ✔ | ✔ | ✔ | ✔ |
| 10% | ✔ | ✔ | ✔ | ✔ |

This indicates that CsA is surprisingly soluble in both GEO and GEO/GMO 10:9 mixture at room temperature and at around physiological temperature. The solubility limit of CsA in these systems was not established and may, therefore be anything above 10 wt%.

### Example 4 - Formation of liquid crystal phase with CsA

Samples of CsA at 5 wt% and 10 wt% in GEO were diluted with isotonic saline and allowed to equilibrate. The phase behaviour was examined between crossed polarisers and under the polarising microscope at 40°C.

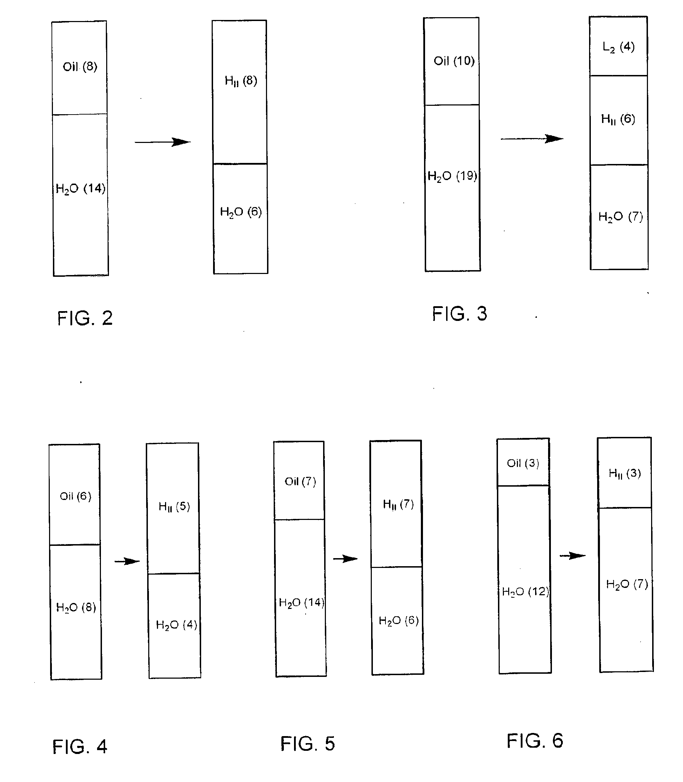

The results are indicated below in Table 2 and shown diagrammatically in Figures 2 - 3. It can be seen that a mixture of 5% CsA in GEO (8 parts) with saline (14 parts) equilibrated to 8 parts reversed hexagonal phase in equilibrium with 6 parts saline. A mixture of 10% CsA in GEO (10 parts) with saline (19 parts) similarly equilibrated to a 3-phase mixture of 4 parts micellar suspension and 6 parts reversed hexagonal phase all in equilibrium with 7 parts saline. All parts are approximate and by volume, relative to the total number of parts in that sample.

**Table 2**

| CsA wt% | Initial | | | Final equilibrium | | |
|---|---|---|---|---|---|---|
| | Parts GEO/CSA | Parts Saline | | Parts L₂ | Parts H_{II} | Parts Saline |
| 5% | 8 | 14 | → | - | 8 | 6 |
| 10% | 10 | 19 | → | 4 | 6 | 7 |

No CsA crystallisation was observed in any sample. From these data it is possible to conclude that the solubility of CsA remains high in structured phases of GEO/water. The data also indicate that CsA induces more negative curvature in the structures and thus tends to favour the formation of L₂ phase in equilibrium with H_{II} and water at higher CsA concentrations.

In detail, Figures 2-3 shows the following:

Figure 2 - GEO with 5% CsA. It can be seen that, despite the CsA content, the sample is characterised by an H_{II} phase in equilibrium with excess water. The H_{II} phase contains approximately 40% water.

Figure 3 - GEO with 10% CsA. Here the higher CsA content induces an L₂-phase. Thus, CsA tends to induce a more negative curvature of the lipid film.

When the temperature of the 10% mixture was reduced, the proportion of L₂ phase was seen to decrease in favour of a greater proportion of H_{II} phase. This indicates that the curvature is also increased by increasing temperature, an observation which is common in such systems.

The effects of temperature were reversible, as tested by heat-cycling the 10% mixture in the polarising microscope and observing the effect of temperature on birefringence

### Example 5 - Mixtures of GEO, GMO and water

The phase behaviour of a mixture of GEO/GMO (10:9) with excess saline was investigated, with and without CsA present. The procedure was as indicated above for Example 4 and the results are shown below in Table 3, and in Figures 4 -6.

**Table 3**

| CsA wt% | Initial | | | Final equilibrium | | |
|---|---|---|---|---|---|---|
| | Parts lipid/CsA | Parts Saline | | Parts L₂ | Parts H_{II} | Parts Saline |
| 0 | 6 | 8 | → | - | 5 | 4 |
| 5% | 7 | 14 | → | - | 7 | 6 |
| 10% | 3 | 12 | → | - | 3 | 7 |

In all cases, the mixture of GEO/GMO/CsA equilibrated to a reversed hexagonal phase in the presence of excess saline.

In detail, Figures 4-6 show the following:

Figure 4 - a mixture of GEO and GMO (10/9) in equilibriuim with excess water is characterised by an H_{II} phase. (Possibly this phase contains somewhat less water than the H_{II} phase from pure GEO.)

Figure 5 - a mixture of GEO and GMO (10/9) containing 5% CsA is characterised by an H_{II} phase.

Figure 6 - a mixture of GEO and GMO (10/9) containing 10% CsA is characterised by an H_{II} phase.

GMO is known to cause somewhat less negative curvature than GEO since the stable phase of GMO in excess water is reversed cubic rather than the reversed hexagonal of GEO. It might therefore be expected that a mixture of GEO and GMO would show less extreme negative curvature that GEO alone. This is supported by the above data indicating that the GEO/GMO mixture did not form the highly negatively curved L₂ phase even in the presence of 10% CsA. The phase behaviour of the composition may thus be further controlled by the appropriate choice of amphiphilic components.

### Example 6 - Preparation of compositions

Compositions comprising ether lipids but without any active agent were prepared to examine their phase behaviour and dispersion properties.

A mixture of 90% GEO and 10% Lutrol F127 (Poloxamer 407 - BASF) was prepared by mixing to form a homogeneous lipid phase. 10 parts by wieght of the lipid mixture was then stirred with 90 parts water. The course dispersion was analysed for structured phases before homogenisation using a microfluidiser. The phase behaviour and structure of this dispersion was then analysed again. The experiment was repeated with varying components as indicated below wherein "NaOl" indicates sodium oleate, "F127" indicates Lutrol F127 and all components are indicated as weight percentage.

**Table 4**

| | GEO % | GMO % | NaOl % | F127 % | H₂O % | Stirred | | Homogenised | |
|---|---|---|---|---|---|---|---|---|---|
| | | | | | | Phase | Structure | Phase | Structure |
| A | 90 | - | - | 10 | - | Homogen | Liquid | - | - |
| B | 9 | - | - | 1 | 90 | Coarse | Hex | Nano | Hex |
| C | 85.5 | - | 4.5 | 10 | - | Homogen | Liquid | - | - |
| D | 8.55 | - | 0.45 | 1 | 90 | Coarse | Hex | Nano | Hex |
| E | 45 | 45 | - | 10 | - | Homogen | Liquid | - | - |
| F | 4.5 | 4.5 | - | 1 | 90 | Coarse | Hex | Nano | Hex |

| | | | | | | | | | |
|---|---|---|---|---|---|---|---|---|---|
| Homogen = Homogeneous solution Coarse = Coarse dispersion Nano = Nanoparticle dispersion Liquid = Unstructured liquid lipid (at melt temperature of 50-60 C) Hex = Reversed hexagonal structure | | | | | | | | | |

### Example 7 - Addition of active agent

The nanoparticle dispersion of composition F prepared in Example 6 (10 ml) is mixed with a solution of the catioic peptide desmopressin (1 ml, 1mg/ml) in deionised water. The solution is then equilibrated for 30-60 minutes.

### Example 8

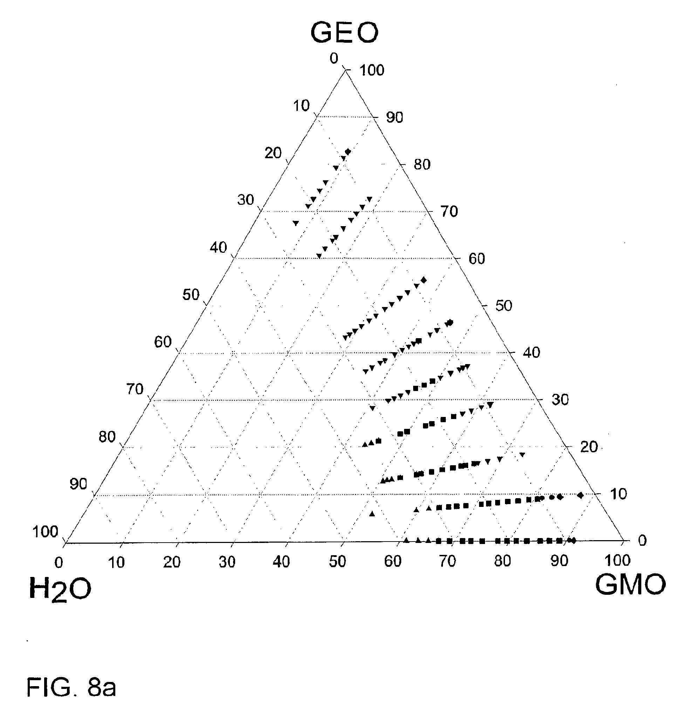

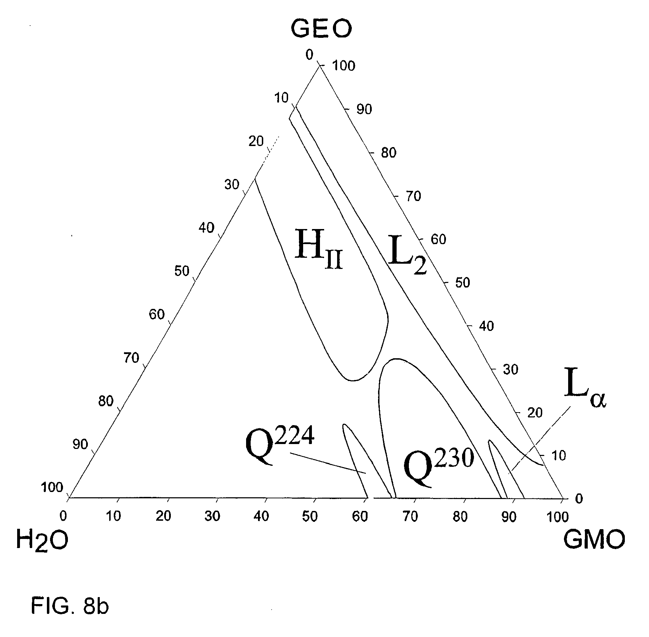

The phase behavior of ternary mixtures of glycerol monooleate (GMO), glyceryl monooleyl ether (GEO) and water was investigated. The samples were prepared by co-melting appropriate amounts of GMO and GEO into the vials, adding water and allowing equilibrate at room temperature for at least 4 weeks before measurements. The resulting mixtures were examined by polarizing microscopy and X-ray diffraction. The phases identified and their location in the ternary phase diagram is shown in Figure 8a. The phases are labeled as follows: diamonds, micellar solution (L₂); circles, lamellar phase (L_{α}); squares, *Iα3d* cubic phase (Q²³⁰); triangles (point upwards), *Pn3m* cubic phase (Q²²⁴); reversed triangles (point downwards), reversed hexagonal phase (H_{II}). Figure 8b shows a schematic phase diagram based on the interpretation of the data in Figure 8a. The results indicate that ternary mixtures form two nonlamellar liquid crystalline phases, Q²²⁴ and H_{II} in equilibrium with water. This opens the possibility to form different liquid crystalline phase dispersions by only changing the composition of the system.

Figure 8a shows identity and location of each phase in the ternary glycerol monooleate (GMO)/glyceryl monooleyl ether (GEO)/water system, as determined by X-ray diffraction.

Figure 8b shows a schematic phase diagram based on the interpretation of the data in Figure 8a.

### Example 9

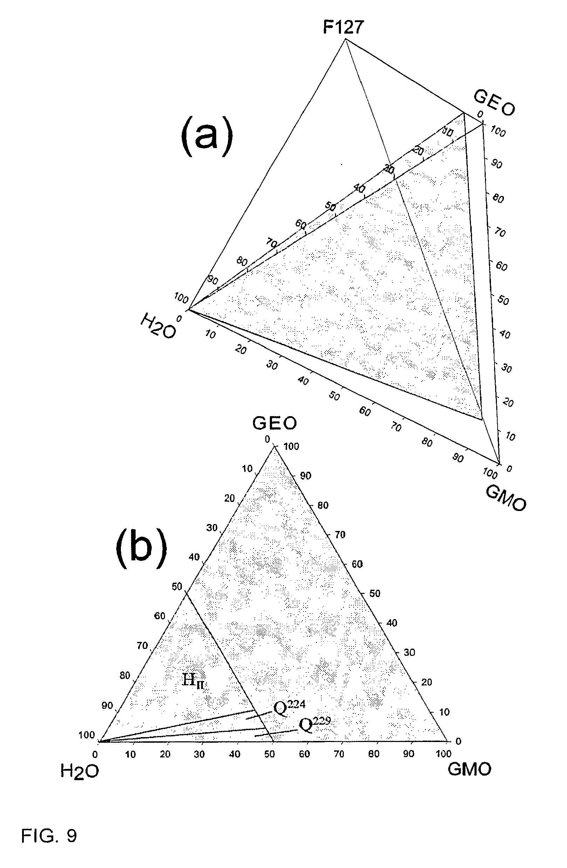

The phase behavior of quaternary mixtures of GMO, GEO, Pluronic F127 and water was investigated. Samples were prepared by co-melting appropriate amounts of GMO and GEO, and adding F127 solution in water into melted lipid mixture to get lipid/polymer ratio of 9/1 (w/w). The total water content was 70 wt% to ensure that the samples will have excess solution phase. The samples were immediately sealed, hand-shaken and left to vortex for at least 2 weeks at room temperature before observation. Figure 9a shows the quaternary composition pyramid and the grey cross-section corresponds to the pseudo-ternary triangle with a fixed lipid-to-polymer ratio of 9/1 in which the samples were investigated. The results reveal that the quaternary mixtures form three different nonlamellar phases in excess solution: two bicontinuous cubic phases, Q²²⁴ and Q²²⁹, and reversed hexagonal phase. It may be concluded that by no more than changing the GEO/GMO ratio it is possible to prepare three types of nonlamellar phase colloidal dispersions which contain different amounts of the ether lipid and have different protection against hydrolytic enzymes, such as lipases.

Figure 9a - location of the GMO/GEO/F127/water system.

Figure 9b - the phases formed in equilibrium with excess water of the GMO/GEO/F127/water system.

### Example 10

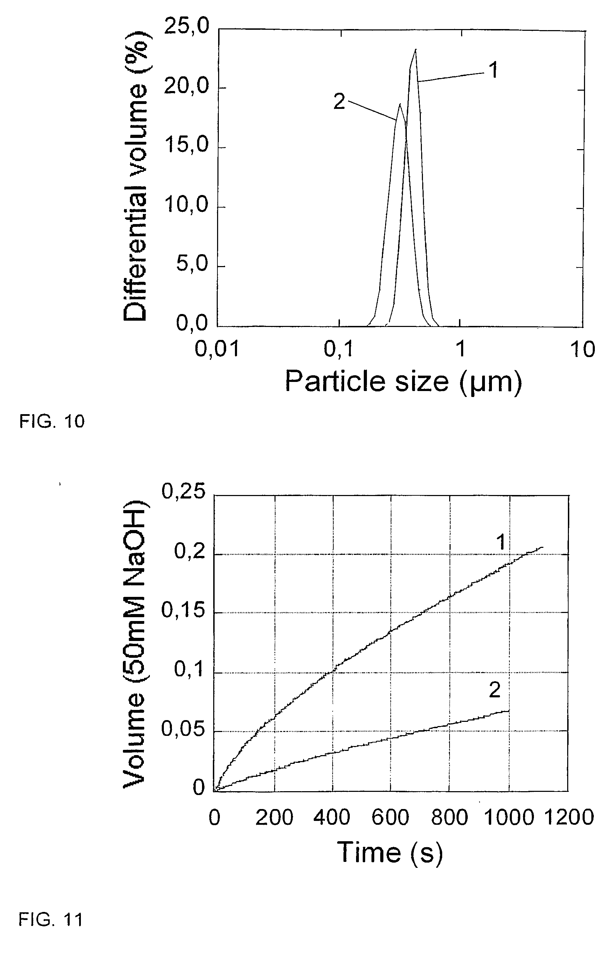

To check the lipase (*Thermomyces lanuginosa*) activity, two colloidal dispersions, GMO/F 127 (9/1 w/w) and GMO/GEO/F127 (GMO/GEO (50/50 w/w), lipid/F127 (9/1 w/w)) were prepared. Samples were prepared by adding GMO or the mixture of GMO and GEO to F127 solution in water, immediately hand-shaking and vortexing the mixture for 24 hours in a mechanical mixing table. The obtained crude dispersions were then homogenized by passing 5 times throught a Microfluidizer 110S (Microfluidics Corp.) at 345 bar pressure and 25°C. The homogenized samples were heat-treated for 20 min at 125°C. Figure 10 shows the particle distributions of the the obtained colloidal dispersions. As may be seen from

Figure 10, both dispersions are characterized by similar sizes of about 350 - 450 nm. The lipase activity on the prepared dispersions was checked by using a pH-stat measurement. In this method the pH-stat titration system monitors pH of the incubation medium and titrates with NaOH any lowering in pH due to the production of the fatty acids from GMO via lipolysis. The pH was set to 6.5 to ensure that fatty acids are in deprotonated form. Therefore, the amount of NaOH added during the lipolysis corresponds to the fatty acids produced and reflects the enzymatic activity on the colloidal dispersions. Figure 11 shows the titration curves of the GMO/F127 (curve 1) and GMO/GEO/F127 (curve 2) dispersions. It indicates that the lipolysis rate is about 3 times lower for the system containing GEO. This example clearly shows that by introduction of non-digestible lipids the dispersions remain much more stable in the presence of hydrolytic enzymes. Therefore, by changing the amount of GEO in the dispersions it is possible to tune the system by getting the desired lipolysis rate and release of the active components incorporated into the nonlamellar particles.

Figure 10 - the particle size distribution of the GMO/F127 (curve 1) and GMO/GEO/F127 (curve 2) dispersions.

Figure 11 - the titration curves of the GMO/F127 (curve 1) and GMO/GEO/F127 curve 2) dispersion.

## Claims

1. A composition suitable for administration to a mammalian subject wherein said composition comprises an active agent and an amphiphilic component comprising at least one amphiphile, wherein at least a portion of said amphiphilic component is an ether-linked lipid comprising a polar group attached to at least one non-polar group by an ether linkage, wherein the composition comprises a non-lamellar phase or forms a non-lamellar phase on contact with an aqueous liquid, wherein said polar group is selected from glycerol, choline, ethanolamine, their esterified, glycosylated and/or phosphated equivalents or a mixture thereof, wherein said non-polar group has 6 to 24 carbon atoms and is of formula -O-CₓH_{y} where y=2x+1 or y=2x+1-2z, where z is the number of unsaturations, and wherein said non-lamellar phase is a normal or reversed liquid crystalline phase or the L₃ phase or any combination thereof.

2. A composition of claim 1 wherein said composition is a particulate composition.

3. A composition as claimed in claim 1 or claim 2 also comprising at least one fatty acid or fatty acid salt.

4. A composition as claimed in any of claims 1 to 3 additionally comprising at least one fragmentation agent.

5. A composition as claimed in any of claims 1 to 4 additionally comprising at least one active agent.

6. A composition as claimed in claim 5 wherein said active agent is at least one agent selected from protein, drug, nutrient, cosmetic, diagnostic, pharmaceutical, vitamin and dietary agents.

7. A composition as claimed in any of claims 1 to 6 wherein the composition is a precursor composition which releases or generates fragments of non-lamellar phase upon contact with an aqueous fluid.

8. A composition as claimed in claim 7 wherein said fragments are generated *in vivo* upon contact with a body fluid.

9. A particulate composition as claimed in any of claims 2 to 8 comprising colloidal particles.

10. A composition as claimed in any of claims 1 to 9 wherein said ether-linked lipid is at least one lipid selected from mono-, di- and tri-glyceride analogues wherein one or more fatty acid residue is replaced by a hydrocarbyl residue, where said hydrocarbyl residues are optionally unsaturated hydrocarbyl groups with 6 to 24 carbon atoms.

11. A composition as claimed in claim 10 wherein said ether lipid comprises glyceryl-1-oleyl ether.

12. A composition as claimed in any of claims 1 to 11 comprising at least 5% by weight ether-linked lipid.

13. A composition as claimed in any of claims 1 to 12 wherein said amphiphilic component comprises ether lipid and ester lipid.

14. A composition as claimed in claim 4 wherein said fragmentation agent is an amphiphilic block copolymer.

15. A method for the production of a particulate composition wherein said composition comprises non-lamellar particles or forms non-lamellar particles on contact with an aqueous fluid, said method comprising forming a mixture comprising an active agent and an amphilic component comprising at least one amphiphile, wherein at least a portion of said amphiphilic component is an ether-linked lipid comprising a polar group attached to at least one non-polar group by an ether linkage, and dispersing said mixture in an aqueous medium wherein said polar group is selected from glycerol, choline, ethanolamine, their esterified, glycosylated and/or phosphated equivalents or a mixture thereof, wherein said non-polar group has 6 to 24 carbon atoms and is of formula -O-CₓH_{y} where y=2x+1 1 or y=2x+1-2z, where z is the number of unsaturations, and wherein said non-lamellar particles comprise a normal or reversed liquid crystalline phase or the L₃ phase or any combination thereof.

16. A method as claimed in claim 15 comprising forming colloidal particles.

17. A method as claimed in claim 15 or claim 16 additionally comprising drying the resulting dispersion.

18. A method as claimed in any of claims 15 to 17 further comprising at least one cycle of heating and cooling.

19. A method as claimed in claim 18 wherein said active agent is loaded by means of said cycle of heating and cooling.

20. A controlled release formulation comprising a composition as claimed in any of claims 1 to 14.

21. A pharmaceutical composition comprising a composition as claimed in any of claims 1 to 14 and at least one pharmaceutically acceptable carrier or excipient.

22. A pharmaceutical composition as claimed in claim 21 in a form selected from creams, gels, eye-drops, aerosols, dispersions, powders, powder filled capsules, liquid filled capsules, tablets, coated tablets, coated capsules, aerosols and aerodynamic powders.

23. A cosmetic formulation comprising a composition as claimed in any of claims 1 to 14.

## Patentansprüche

1. Zusammensetzung, die zur Verabreichung an ein Säugetiersubjekt geeignet ist, wobei die Zusammensetzung einen Wirkstoff und einen amphiphilen Bestandteil umfasst, der mindestens ein Amphiphil umfasst, wobei mindestens ein Teil des amphiphilen Bestandteils ein etherverknüpftes Lipid ist, das eine polare Gruppe umfasst, die an mindestens einer nichtpolaren Gruppe durch eine Etherbindung befestigt ist, wobei die Zusammensetzung eine nichtlamellare Phase umfasst oder bei Kontakt mit einer wässrigen Flüssigkeit eine nichtlamellare Phase bildet, wobei die polare Gruppe ausgewählt ist aus Glycerol, Cholin, Ethanolamin, deren esterifizierten, glykosylierten und/oder phosphatierten Äquivalenten oder eines Gemischs davon, wobei die nichtpolare Gruppe 6 bis 24 Kohlenstoffatome besitzt und Formel -O-CₓH_{y} aufweist, wobei y = 2x + 1 oder y = 2x + 1 - 2z ist, wobei z die Anzahl der Nichtsättigungen darstellt und wobei die nichtlamellare Phase eine flüssigkristalline Normal- oder Umkehrphase oder die L₃-Phase oder eine beliebige Kombination davon ist.

2. Zusammensetzung nach Anspruch 1, wobei die Zusammensetzung eine teilchenförmige Zusammensetzung ist.

3. Zusammensetzung nach Anspruch 1 oder 2, die auch mindestens eine Fettsäure oder ein Fettsäuresalz umfasst.

4. Zusammensetzung nach einem der Ansprüche 1 bis 3, die zusätzlich mindestens ein Fragmentationsmittel umfasst.

5. Zusammensetzung nach einem der Ansprüche 1 bis 4, die zusätzlich mindestens einen Wirkstoff umfasst.

6. Zusammensetzung nach Anspruch 5, wobei der Wirkstoff mindestens ein Mittel ist, ausgewählt aus Protein, Arzneimittel, Nährstoff, kosmetischem Mittel, diagnostischem Mittel, pharmazeutischem Mittel, Vitamin und diätetischem Mittel.

7. Zusammensetzung nach einem der Ansprüche 1 bis 6, wobei die Zusammensetzung eine Vorläuferzusammensetzung ist, welche bei Kontakt mit einer wässrigen Flüssigkeit Fragmente der nichtlamellaren Phase freisetzt oder erzeugt.

8. Zusammensetzung nach Anspruch 7, wobei die Fragmente *in vivo* bei Kontakt mit einer Körperflüssigkeit gebildet werden.

9. Teilchenzusammensetzung nach einem der Ansprüche 2 bis 8, die kolloidale Teilchen umfasst.

10. Zusammensetzung nach einem der Ansprüche 1 bis 9, wobei das etherverknüpfte Lipid mindestens ein Lipid ist, ausgewählt aus Mono-, Di- und Triglycerid-Analoga, wobei ein oder mehrere Fettsäurerest(e) durch einen Hydrocarbylrest ersetzt sind, wobei die Hydrocarbylreste optional nichtgesättigte Hydrocarbylgruppen mit 6 bis 24 Kohlenstoffatomen sind.

11. Zusammensetzung nach Anspruch 10, wobei das Etherlipid Glyceryl-1-oleyl-ether umfasst.

12. Zusammensetzung nach einem der Ansprüche 1 bis 11, die mindestens 5 Gewichtsprozent etherverknüpftes Lipid umfasst.

13. Zusammensetzung nach einem der Ansprüche 1 bis 12, wobei der amphiphile Bestandteil Etherlipid und Esterlipid umfasst.

14. Zusammensetzung nach Anspruch 4, wobei das Fragmentationsmittel ein amphiphiles Blockcopolymer ist.

15. Verfahren zur Herstellung einer Teilchenzusammensetzung, wobei die Zusammensetzung nichtlamellare Teilchen umfasst oder bei Kontakt mit einer wässrigen Flüssigkeit nichtlamellare Teilchen bildet, wobei das Verfahren die Bildung eines Gemischs, das einen Wirkstoff und einen amphiphilen Bestandteil umfasst, der mindestens ein Amphiphil umfasst, wobei mindestens ein Teil des amphiphilen Bestandteils ein etherverknüpftes Lipid ist, das eine polare Gruppe umfasst, die an mindestens einer nichtpolaren Gruppe durch eine Etherverbindung befestigt ist, sowie das Dispergieren des Gemischs in einem wässrigen Medium umfasst, wobei die polare Gruppe ausgewählt ist aus Glycerol, Cholin, Ethanolamin, deren esterifizierten, glykosylierten und/oder phosphatierten Äquivalenten oder einem Gemisch davon, wobei die nichtpolare Gruppe 6 bis 24 Kohlenstoffatome besitzt und die Formel -O-CₓH_{y} aufweist, wobei y = 2x + 1 oder y = 2x + 1 - 2z ist, wobei z die Anzahl der Nichtsättigungen darstellt und wobei die nichtlamellaren Teilchen eine flüssigkristalline Normal- oder Umkehrphase oder die L₃-Phase oder eine beliebige Kombination davon umfassen.

16. Verfahren nach Anspruch 15, das die Bildung von kolloidalen Teilchen umfasst.

17. Verfahren nach Anspruch 15 oder 16, das zusätzlich das Trocknen der entstehenden Dispersion umfasst.

18. Verfahren nach einem der Ansprüche 15 bis 17, das ferner mindestens einen Zyklus aus Erhitzen und Abkühlen umfasst.

19. Verfahren nach Anspruch 18, wobei der Wirkstoff mithilfe des Zyklus aus Erhitzen und Abkühlen geladen wird.

20. Retardformulierung, die eine Zusammensetzung nach einem der Ansprüche 1 bis 14 umfasst.

21. Pharmazeutische Zusammensetzung, die eine Zusammensetzung nach einem der Ansprüche 1 bis 14 und mindestens ein pharmazeutisch anerkanntes Trägermittel oder einen Hilfsstoff umfasst.

22. Pharmazeutische Zusammensetzung nach Anspruch 21 in einer Form, die aus Cremes, Gelen, Augentropfen, Aerosolen, Dispersionen, Pulvern, mit Pulver gefüllten Kapseln, mit Flüssigkeit gefüllten Kapseln, Tabletten, überzogenen Tabletten, überzogenen Kapseln, Aerosol- und aerodynamischen Pulvern ausgewählt ist.

23. Kosmetische Formulierung, die eine Zusammensetzung nach einem der Ansprüche 1 bis 14 umfasst.

## Revendications

1. Composition convenant à une administration à un sujet mammifère, dans laquelle ladite composition comprend un agent actif et un composant amphiphile comprenant au moins un amphiphile, dans laquelle au moins une portion dudit composant amphiphile est un lipide à liaison éther comprenant un groupement polaire fixé à au moins un groupement non polaire par une liaison éther, dans laquelle la composition comprend une phase non lamellaire ou forme une phase non lamellaire par contact avec un liquide aqueux, dans laquelle ledit groupement polaire est choisi parmi le glycérol, la choline, l'éthanolamine, leurs équivalents estérifiés, glycosylés et/ou phosphatés ou un de leurs mélanges, dans laquelle ledit groupement non polaire présente 6 à 24 atomes de carbone et a pour formule -O-CₓH_{y}, où y=2x+1 ou y=2x1-2z, où z est le nombre d'insaturations, et dans laquelle ladite phase non lamellaire est une phase cristalline liquide normale ou inversée ou bien la phase L₃ ou encore une combinaison quelconque de celles-ci.

2. Composition selon la revendication 1, dans laquelle ladite composition est une composition particulaire.

3. Composition selon la revendication 1 ou la revendication 2, comprenant également au moins un acide gras ou un sel d'acide gras.

4. Composition selon l'une quelconque des revendications 1 à 3, comprenant en outre au moins un agent de fragmentation.

5. Composition selon l'une quelconque des revendications 1 à 4, comprenant en outre au moins un agent actif.

6. Composition selon la revendication 5, dans laquelle ledit agent actif est au moins un agent choisi parmi des agents de types protéine, médicament, aliment, cosmétique, diagnostique, pharmaceutique, vitamine et diététique.

7. Composition selon l'une quelconque des revendications 1 à 6, dans laquelle la composition est une composition précurseur qui libère ou génère des fragments de phase non lamellaire lors d'un contact avec un fluide aqueux.

8. Composition selon la revendication 7, dans laquelle lesdits fragments sont générés in vivo lors d'un contact avec un fluide corporel.

9. Composition particulaire selon l'une quelconque des revendications 2 à 8, comprenant des particules colloïdales.

10. Composition selon l'une quelconque des revendications 1 à 9, dans laquelle ledit lipide à liaison éther est au moins un lipide choisi parmi des analogues mono-, di- et triglycéridiques, dans laquelle un ou plusieurs résidus d'acides gras est ou sont remplacés par un résidu hydrocarbyle, où lesdits résidus hydrocarbyle sont des groupements hydrocarbyle éventuellement insaturés ayant 6 à 24 atomes de carbone.

11. Composition selon la revendication 10, dans laquelle ledit lipide d'éther comprend un éther de glycéryl-1-oléyle.

12. Composition selon l'une quelconque des revendications 1 à 11, comprenant au moins 5 % en poids de lipide à liaison éther.

13. Composition selon l'une quelconque des revendications 1 à 12, dans laquelle ledit composant amphiphile comprend un lipide d'éther et un lipide d'ester.

14. Composition selon la revendication 4, dans laquelle ledit agent de fragmentation est un copolymère séquencé amphiphile.

15. Procédé pour la production d'une composition particulaire, dans lequel ladite composition comprend des particules non lamellaires ou forme des particules non lamellaires par contact avec un fluide aqueux, ledit procédé comprenant la formation d'un mélange comprenant un agent actif et un composant amphiphile comprenant au moins un amphiphile, dans lequel au moins une portion dudit composant amphiphile est un lipide à liaison éther comprenant un groupement polaire fixé à au moins un groupement non polaire par une liaison éther, et la dispersion dudit mélange dans un milieu aqueux, dans lequel ledit groupement polaire est choisi parmi le glycérol, la choline, l'éthanolamine, leurs équivalents estérifiés, glycosylés et/ou phosphatés et/ou un de leurs mélanges, dans lequel ledit groupement non polaire présente 6 à 24 atomes de carbone et a pour formule -O-CₓH_{y}, où y=2x+1 ou y=2x+1-2z, où z est le nombre d'insaturations, et dans lequel lesdites particules non lamellaires comprennent une phase cristalline liquide normale ou inversée ou la phase L₃ ou encore une combinaison quelconque de celles-ci.

16. Procédé selon la revendication 15, comprenant la formation de particules colloïdales.

17. Procédé selon la revendication 15 ou la revendication 16, comprenant en outre le séchage de la dispersion obtenue.

18. Procédé selon l'une quelconque des revendications 15 à 17, comprenant en outre au moins un cycle de chauffage et de refroidissement.

19. Procédé selon la revendication 18, dans lequel ledit agent actif est chargé au moyen dudit cycle de chauffage et de refroidissement.

20. Formulation à libération contrôlée comprenant une composition selon l'une quelconque des revendications 1 à 14.

21. Composition pharmaceutique comprenant une composition selon l'une quelconque des revendications 1 à 14 et au moins un véhicule ou un excipient pharmaceutiquement acceptable.

22. Composition pharmaceutique selon la revendication 21 sous une forme choisie parmi des crèmes, des gels, des gouttes pour les yeux, des aérosols, des dispersions, des poudres, des capsules remplies de poudre, des capsules remplies de liquide, des comprimés, des comprimés enrobés, des capsules enrobées, des aérosols et des poudres aérodynamiques.

23. Formulation cosmétique comprenant une composition selon l'une quelconque des revendications 1 à 14.
